Europäisches Patentamt

European Patent Office

Office européen des brevets

(11) Veröffentlichungsnummer: **0 164 119**
A2

## (12) EUROPÄISCHE PATENTANMELDUNG

(21) Anmeldenummer: 85107001.1

(22) Anmeldetag: 05.06.85

(51) Int. Cl.⁴: **G 01 N 33/62,** C 12 Q 1/58, **G 01 N 33/04**

(30) Priorität: 06.06.84 DE 3420987

(43) Veröffentlichungstag der Anmeldung: 11.12.85 Patentblatt 85/50

(84) Benannte Vertragsstaaten: **AT BE CH DE FR GB LI NL SE**

(71) Anmelder: **Arnstadt, Klaus-Ingo, Dr., Waldvögeleinstrasse 4a, D-8000 München 50 (DE)**

(72) Erfinder: **Arnstadt, Klaus-Ingo, Dr., Waldvögeleinstrasse 4a, D-8000 München 50 (DE)**

(74) Vertreter: **Fürniss, Peter, Dr., Am Büchl 15, D-8050 Freising (DE)**

(54) **Bestimmung des Harnstoffgehaltes von Milch.**

(57) Bei der kolorimetrischen Harnstoffbestimmung in Milch wird die Eignung der Milchprobe durch Ermittlung des Blindwertes nach Abbau des Harnstoffs mittels Urease festgestellt. Bei Blindwerten über 5 mg/100 ml ist die Milchprobe ungeeignet.

## Beschreibung

Die Erfindung betrifft ein Verfahren zur quantitativen Bestimmung von Harnstoff in Milch durch Umsetzung des Harnstoffs mit einem Aldehyd und kolorimetrische Bestimmung des Umsetzungsproduktes. Der Harnstoffgehalt der Milch ist zusammen mit dem Eiweißgehalt ein Indikator der Versorgungslage der Kühe. Ein zu hoher Gehalt an Harnstoff beruht im allgemeinen auf zu hoher Protein- und/oder zu niedriger Energieversorgung, was zur Abnahme der Milchleistung und zu Fruchbarkeitsstörungen führen kann (Feddersen, Tierzüchter 36, S. 71-72 (1984) ). Es wird festgestellt, daß ein geeignetes Verfahren für die Routinemessung des Harnstoffgehaltes bisher noch fehlt.

In: Milchwissenschaft 37 (1) S. 6-9 (1982) weist Kaufmann auf die Bedeutung der Harnstoff-Messung als Fütterungskontrolle hin.

0164119

Timm, Gravert und Pabst beschreiben in: Kieler milchwirtschaftliche Forschungsberichte, 33 (3) S. 213-220 (1981) ein Verfahren zur Harnstoffbestimmung in der Milch. Danach wird die Probe zunächst vom Eiweiß befreit und nach Zugabe von p-Dimethylaminobenzaldehyd der Gehalt an Harnstoff im Filtrat durch Messung der Extinktion bei einer Wellenlänge von 420 nm bestimmt. Die Wiederholungsmessung für Harnstoff ergab eine Genauigkeit von r = 0,70 , während die Wiederholbarkeit für Parallelproben r = 0,88 betrug.

Von der Industrie wurden Verfahren zur Bestimmung von Harnstoff in Milch auf den Markt gebracht. In der Firmenschrift: „Harnstoff/Ammoniak" der Boehringer Mannheim GmbH wird die Bestimmung von Harnstoff in Milch nach Ausfällen der enthaltenen Proteine und Neutralisieren der überstehenden klaren Lösung durch Spaltung des Harnstoffs in Gegenwart des Enzyms Urease zu Ammoniak und Kohlendioxyd und Umsetzung des erhaltenen Ammoniaks zu d-Ketoglutarat in Gegenwart von Glutamat-Dehydrogenase und reduziertem Nicotinamid-adenin-dinucleotid zu L-Glutamat durchgeführt. Dieses Verfahren ist zwar zuverlässig und führt zu genauen Ergebnissen. Die Messung ist jedoch sehr aufwendig. So sind z.B. mehrere Pipettierschritte erforderlich, so daß das Verfahren für Serienmessungen nicht geeignet ist.

In: „diagnostica dialog" 1/1983 der Boehringer Mannheim GmbH wird auf S. 20, 21 ein Verfahren zur Bestimmung der Harnstoffkonzentration in Milch und Körperflüssigkeiten bei Hochleistungskühen mit dem Teststreifen Reflotest® Urea und dem Reflexionsphotometer Reflomat® beschrieben. Hierbei wird der Harnstoff mit dem Ferment Urease zu Ammoniak abgebaut, der mit Neßlers Reagens kolorimetrisch bestimmt wird. Dies Verfahren besitzt jedoch eine zu geringe Empfindlichkeit, da nur Werte von $\geq 30$ mg/dl ange- zeigt werden. Deshalb wird empfohlen, auf andere Testverfahren auszuweichen, wenn genaue Resultate bei niedrigen Harnstoff-Konzentrationen erzielt werden sollen. Eine andere Methode erscheint besonders wünschenswert, da der ideale physiologische Normbereich bei 15-20mg/dl liegt.

Die Bestimmung des Harnstoffgehaltes von Milch kann grundsätzlich zu falschen Ergebnissen führen, da die Korrelation des Harnstoffgehaltes der Milch zum Gehalt des Serums an Harnstoff und damit zum Ernährungszustand der Kuh bei klinischer und subklinischer Euterentzündung (Mastitis) nicht mehr gegeben ist. Insbesondere bei gealterten oder sogar sauren Milchproben lassen die kolorimetrisch gefundenen Harnstoffwerte keinen Rückschluß auf den Harnstoffgehalt des Serums und den Ernährungszustand der Kuh zu. Für die Praxis ist es von ausschlaggebender Bedeutung, geeignete frische Milchproben von ungeeigneten gealterten Milchproben bei der kolorimetrischen Harnstoffbestimmung unterscheiden zu können.

Der Erfindung liegt daher die Aufgabe zugrunde, für die Bestimmung von Harnstoff in Milch ein Verfahren zur Verfügung zu stellen, bei dem die genannten Störungen erkannt werden und fehlerhafte Harnstoffwerte ausgeschieden werden können.

Diese Aufgabe wird bei einem Verfahren der eingangs genannten Gattung dadurch gelöst, daß man den Harnstoffgehalt der Milch mit einem Aldehyd, der mit Harnstoff ein farbiges Reaktionsprodukt bildet, kolorimetrisch ermittelt und zur Feststellung der Eignung der Milch in einer Kontrollprobe nach Abbau des Harnstoffs mittels Urease den Blindwert mit einem Aldehyd, der außer mit Harnstoff auch mit anderen amino- bzw. amidgruppenhaltigen Verbindungen einer Schiff'schen Base-Reaktion ein farbiges Reaktionsprodukt bildet, kolorimetrisch ermittelt.

Die Reaktionen können in an sich üblicher Weise in Reagensgläsern oder Küvetten durchgeführt werden.

- 4 -

Bei einer Ausführungsform des erfindungsgemäßen Verfahrens wird das Eiweiß mit einer wäßrigen Trichloressigsäurelösung entfernt, der Aldehyd, der mit Harnstoff ein farbiges Reaktionsprodukt bildet, zugegeben und der Harnstoffgehalt kolorimetrisch bestimmt.

Zur Feststellung der Eignung der Milch wird eine Kontrollprobe mit Urease behandelt, wodurch der Harnstoff abgebaut wird. Ein Aldehyd, der außer mit Harnstoff auch mit anderen amino- bzw. amidgruppenhaltigen Verbindungen in einer Schiff'-schen Base-Reaktion ein farbiges Reaktionsprodukt bildet, wird zur Kontrollprobe zugesetzt und die auftretende Färbung kolorimetrisch ermittelt.

Bei einer weiteren Ausführungsform wird das erfindungsgemäße Verfahren als Eintopfreaktion durchgeführt.Dabei gibt man zu der Milchprobe bzw. Kontrollprobe eine Vormischung von Fällungsmittel und Farbreagens, mischt erneut und zentrifugiert 5 min bei 2000 bis 6000 r.p.m. . Die Färbung des Überstandes wird direkt photometrisch gemessen.

Man kann die beschriebene kolorimetrische Harnstoffbestimmung von Milch- und Kontrollproben auch auf Papier oder einem festen Träger durchführen, wobei naturgemäß der Zentrifugationsschritt entfällt. Die Auswertung kann z.B. mit Hilfe eines Reflexionsphotometers oder visuell erfolgen.

Das erfindungsgemäße Verfahren gibt zuverlässige Aussagen über den Ernährungszustand der Kuh. Fehlerhafte Harnstoffwerte werden sofort erkannt und können daher nicht zu Fehlschlüssen über den Ernährungszustand der Kuh führen. Das vorgeschlagene Verfahren ist wegen seiner einfachen und billigen Durchführung insbesondere für Harnstoffbestimmungen in Serien geeignet, wie sie in der Praxis ständig vorkommen. Das Verfahren ist einfach und schnell durchzuführen.

0164119

Es sind nur wenige Pipettierschritte notwendig; insgesamt ist die Reaktion innerhalb drei Minuten beendet. Bei der Ausführungsform mittels Papier oder eines sonstigen Trägers entfallen die Zentrifugierschritte vollständig.

Da das erfindungsgemäße Verfahren nicht ammoniakempfindlich ist, werden die Ergebnisse im Gegensatz zu anderen Verfahren nicht verfälscht durch den Gehalt der Atmosphäre an Ammoniak, das bekanntlich sowohl in der Laboratoriums- als auch in der bäuerlichen Praxis im Viehstall vorkommt. Für andere Analysen, z.B. für die Progesteronbestimmung auf der Basis eines Enzym-Immuntests (EIA) darf die Milch keine Konservierungsstoffe enthalten. Wenn in solchen Fällen auch der Harnstoffgehalt der Milch bestimmt werden soll, ist es unbedingt notwendig, den Aussagegehalt der kolorimetrisch erhaltenen Harnstoffwerte nach dem erfindungsgemäßen Verfahren zu überprüfen.

Als Aldehyd für die Schiff'sche Base-Reaktion mit Harnstoff zu einem farbigen Reaktionsprodukt eignen sich zahlreiche aromatische Aldehyde, z.B. 4-Dimethylamino-zimtaldehyd, o-Phthalaldehyd oder Salicylaldehyd. Besonders geeignet und daher bevorzugt zur Durchführung des erfindungsgemäßen Verfahrens bei der Ausführungsform in Reagensröhrchen oder Küvetten ist der 4-Dimethylaminobenzaldehyd (p-DMAB). Wird ein fester Träger, z.B. Papier, verwendet, ist 4-Dimethylamino-zimtaldehyd bevorzugt.

An Milchproben kann Vor-, Haupt-, Nach- und Gesamtgemelk eingesetzt werden. Die Milchprobe sollte nicht länger als ein bis zwei Tage bei Raumtemperatur, am besten nur einen Tag bei 4 bis 8 $^\circ$C im Kühlschrank aufgehoben werden. Eine langfristige Lagerung der Milch bei -20 $^\circ$C ist möglich. Zur Durchführung der Bestimmung werden nur geringe Mengen von ca. 0,001 bis 2ml je nach Ausführungsform benötigt.

Bei der Durchführung der Reaktion in Reagensgläsern bzw. Küvetten werden die Milchproben (0,5 bis 10ml) zu Beginn bei 3000 - 5000 r.p.m. zentrifugiert. Ein Aliquot jeder Probe wird mit einer Lösung des Enzyms Urease für die Blindwertmessung verwendet, während ein anderes Aliquot direkt der kolorimetrischen Harnstoffbestimmung dient. Zu den Proben wird Trichloressigsäure zur Ausfällung des Eiweiß zugegeben. Nach dem Zentrifugieren wird die überstehende Lösung mit dem Farbreagens versetzt. Die Färbung ist mehr als eine Stunde lang stabil. Die Extinktion wird im Photometer bei 436 nm gemessen. Zu Beginn der Messung wird die Extinktion (bzw. deren Differenz zu einem Leerwert) einer Lösung bekannter Harnstoff-Konzentration ("Standardlösung") ermittelt.

Aus der Extinktion der Standardlösung wird die Eichgerade gezeichnet. Der Harnstoffgehalt der Proben und Blindwerte wird aus der Eichkurve abgelesen. Ist am Photometer ein Rechner angeschlossen, kann die manuelle Auswertung mit Hilfe der gezeichneten Eichkurve entfallen, wenn ein entsprechendes Rechenprogramm eingegeben wird. Die Meßergebnisse werden dann direkt als Harnstoffgehalt der Proben ausgedruckt.

Noch einfacher kann aus der gemessenen Extinktion der bekannten Harnstoff-Konzentration einer Standardlösung ein Faktor zur Multiplikation mit den Extinktionen (bzw. deren Differenz zu einem Leerwert der Harnstoff-Konzentration 0) der Proben erstellt werden, dessen Anwendung direkt den Harnstoffgehalt der Milchproben ergibt.

Die Blindwerte werden von den erhaltenen Werten der Proben subtrahiert. Milchproben mit einem Blindwert $> 5$mg/100ml sind erfahrungsgemäß für eine Harnstoffbestimmung ungeeignet. Die Blindwerte geeigneter Milchproben betragen im allgemeinen 2 bis 3mg/100ml.

Von den üblichen Konservierungsmitteln für Milchproben stört Bichromat wegen seiner Eigenfärbung die Harnstoffbestimmung mit p-DMAB. Ein Borsäure/Sorbinsäure-Gemisch stört nur die Blindwertmessung. Natriumazid stört diese Harnstoffbestimmung zwar nicht, ist aber als Konservierungsmittel nur einsetzbar, wenn die Milchprobe nicht für andere Analysen benutzt wird, wo es stört, wie z.B. bei einem Enzym-Immuntest für Progesteron.

Beispiel 1:

Testausführung: 2ml Milch jeder Kuh werden in einem Röhrchen 5 min lang bei 4000 r.p.m. zentrifugiert. Je 0,5 ml der unterstehenden fettfreien Phase werden in je ein Röhrchen mit und ohne 10 I.U. Urease übertragen. Nach 5 min Raumtemperatur wird 1 ml 18%iger Trichloressigsäure zugegeben. Nach dem Mischen wird 5 min lang bei 4000 r.p.m. zentrifugiert. Zu 0,5 ml des Überstandes werden 0,5 ml salzsaurer äthanolischer p-Dimethylaminobenzaldehyd-Lösung (20g/l) gegeben und gemischt. Nach 3 bis 60 min wird die Extinktion bei 435 nm gemessen.

Auswertung: Bei Messung gegen einen Standard ( Harnstofflösung, 60 mg/100ml) wird die Konzentration der Proben durch Multiplizieren der gemessenen Extinktionsdifferenz mit dem folgenden Faktor errechnet:

$$c = \Delta E_{Probe} \times \frac{60}{\Delta E_{Standard}}$$

Die Proben wurden nach Eingang bis zu acht Tagen bei Raumtemperatur stehengelassen; sie wurden zu Beginn und alle zwei Tage gemessen. In der folgenden Tabelle sind in der ersten Zeile die Namen der Kühe, in der ersten Spalte der Zeitpunkt der Messung des Harnstoffgehalts angeführt.

Der Meßbereich betrug 0 bis 60mg Harnstoff/100ml Milch; die Variationskoeffizienten betrugen innerhalb der Testserie 2,5% (n=20), von Test zu Test 4,3% (n=14); die Wiederfindung war 98,5% (n=9, VK=1,6%, r=0,9996). Die Korrelation zu einer enzymatischen Vergleichsmethode aus der Lebensmittelanalytik, die als die genaueste Harnstoffbestimmungsmethode eingestuft wurde, betrug r=0,986 (n=30) und r=0,962 (n=10).

Aus den erhaltenen Werten läßt sich entnehmen, daß am Tag 0 sämtliche Milchproben bis auf eine Ausnahme zur kolorimetrischen Harnstoffbestimmung geeignet waren. Die Blindwerte sind niedrig (zwischen 3,0 und 5,0) und der kolorimetrisch erhaltene Wert 1) steht nach Abzug des Blindwertes 2) in guter Übereinstimmung mit dem enzymatisch erhaltenen Vergleichswert 3). Aus dem hohen Blindwert von 11,5 am Tag 0 für die Kuh Omega ersieht man sofort, daß diese Milch zur Harnstoff-Harnstoffwert 20,5 ergäbe nach Abzug des Blindwertes 11,5 den viel zu niedrigen Wert 9,0.

Aufgrund der hohen Blindwerte am Tag 2 sind die Analysen für die Kühe Lotte, Blatine , Kastanie und Lia ebenfalls ungeeignet, während am vierten Tag überhaupt keine Milchprobe mehr für die Harnstoffbestimmung geeignet ist. Weiterhin entnimmt man der Tabelle, daß die Milchproben der Kühe 70021023 und Gabi Bakterien enthalten, die Harnstoff abbauen. Daher erhält man bei den vier Tage alten Proben selbst bei enzymatischer Bestimmung Fehlwerte.

Tabelle 1

| Tag | Kuh | Flocke | Flora | 70021023 | Omega | Flicka | Lotte | Blatine | Kastanie | Gabi | Lia |
|---|---|---|---|---|---|---|---|---|---|---|---|
| 0 | 1) | 30 | 42,5 | 30 | 20,5 | 26,5 | 21 | 19 | 21 | 45,5 | 39,5 |
|  | 2) | 3,5 | 5,0 | 3,5 | 11,5 | 4,8 | 3 | 3,5 | 3,5 | 4,8 | 4,8 |
|  | 3) | 23,5 | 36 | 27,4 | 15,6 | 21,9 | 18,2 | 14,2 | 16,9 | 42 | 35,1 |
| 2 | 1) | 28,5 | 43 | 26 | 22,5 | 27 | 21 | 18 | 18,5 | 45 | 42,5 |
|  | 2) | 1,5 | 4,5 | 3,0 | 18,5 | 5,0 | 17 | 14 | 12 | 6,0 | 12,0 |
|  | 3) | 23 | 34,2 | 20,9 | 14,0 | 20,2 | 15,8 | 14,6 | 14,1 | 38,6 | 33,9 |
| 4 | 1) | 30,5 | 42 | 21 | 24 | 27 | 21 | 14,5 | 17 | 51,5 | 49 |
|  | 2) | 20,5 | 41 | 21 | 21,5 | 25,5 | 20 | 14 | 16 | 40 | 46,5 |
|  | 3) | 19,7 | 35,4 | 17,1 | 14,4 | 19,4 | 11,6 | 11,6 | 14,2 | 31,4 | - |
| 6 | 1) | 29,5 | 40,5 | 19,5 | 25 | 24 | 17 | 11 | 15 | 64 | 56 |
|  | 2) |  |  |  |  |  |  |  |  | 52 |  |
|  | 3) | 18,2 | 30,9 | 12,4 | 14,8 | 17,4 | 12 | 7,5 | 10,9 | 29,5 | 31,2 |
| 8 | 1) | 30,5 | - | - | 28 | 24 | 13 | 12 | 16,5 | 65 | 57 |
|  | 2) |  |  |  |  |  |  |  |  | 57 |  |
|  | 3) | 14,8 | 24,2 | - | 15,1 | 14,9 | 7,4 | 9,7 | 7,5 | 25,4 | 26,1 |

1)    kolorimetrischer Test ohne Blindwertkorrektur
2)          "          Blindwert
3)    enzymatischer Test (Vergleich)

Beispiel 2:
_____

Je 0,2 ml der fettfreien Phase einer Milchprobe werden in Reaktionsgefäße pipettiert, von denen das eine 10 I.U. Ureaselösung enthält. Nach 5 min bei Raumtemperatur wird 1 ml einer Vormischung (Verhältnis 1:1) von Trichloressigsäurelösung (18 %ig) und p-DMAB-Lösung (20g/l) hinzugegeben, gemischt und 5 min bei 5000 r.p.m. zentrifugiert. Die Messung der Extinktion erfolgt direkt aus dem Reaktionsgefäß, wobei der Überstand mit Hilfe eines Durchflußküvettensystems abgesaugt wird.

Ergebnisse:

Standardlösung: Extinktionsdifferenz E gegen $H_2O$ gemessen:

|        | E     |
|--------|-------|
| 0  mg/dl | 0,110 |
| 60 mg/dl | 0,518 |

Milchproben: Extinktionsdifferenz $\Delta E$ gegen 0-Standard gemessen: sh. Tabelle 2.

## Tabelle 2

| Milchprobe Nr. | $\Delta E$ | Harnstoff-konzentration mg/dl | Vergleichsmessg. nach Methode Bsp. 1 | Vergleich Bsp.2 gegen 1 in % |
|---|---|---|---|---|
| 1 | 0,137 | 20,1 | 20,3 | 99,0 |
| 2 | 0,202 | 29,7 | 32,5 | 91,4 |
| 3 | 0,175 | 25,7 | 26,1 | 98,5 |
| 4 | 0,190 | 27,9 | 27,7 | 100,7 |
| 5 | 0,139 | 20,4 | 19,1 | 106,8 |
| 6 | 0,213 | 31,3 | 28,7 | 109,0 |
| 7 | 0,232 | 34,1 | 32,1 | 106,2 |

Beispiel 3:

Je 5 µl der Milchprobe bzw. der Harnstoff-Standardlösung werden auf Filterpapier (Tüpfelplatte bzw. Mikrotiterplatte) aufgetragen. Bei den Kontrollproben werden 1 I.U. Urease in üblicher Pufferlösung hinzugefügt. Nach 3 min bei Raumtemperatur erfolgt die Zugabe von 5 µl einer Reagenzlösung wie in Beispiel 1, wobei als Farbreagens p-DMAB bzw. 4-Dimethylaminozimtaldehyd benutzt wird. Die Beurteilung der Harnstoffkonzentration erfolgt durch visuellen Vergleich der Farbintensität nach einer bestimmten Zeit (ca. 3 min) mit der Färbung von parallel aufgetragenen Standardlösungen.

Tabelle 3

Auswertung des kolorimetrischen Harnstoff-Tests auf dem festen Träger Filterpapier (Farbreagens 4-Dimethylaminozimtaldehyd)

| Standardlösungen mg Harnstoff/dl | Färbung |
|---|---|
| 0 | gelblich |
| 10 | blaß rosa |
| 20 | schwach rosa |
| 30 | rosa· |
| 40 | dunkelrosa |

Die in diesem Beispiel visuell beurteilten Harnstoffwerte der Milchprobe stimmten gut mit den quantitativ wie in Beispiel 1 gemessenen Harnstoffkonzentrationen überein.

0164119

Patentansprüche
————————————

1. Verfahren zur quantitativen Bestimmung von Harnstoff in Milch durch Umsetzung des Harnstoffes mit einem Aldehyd und kolorimetrische Bestimmung des Umsetzungsproduktes, dadurch g e k e n n z e i c h n e t , daß man den Harnstoffgehalt der Milch mit einem Aldehyd , der mit Harnstoff ein farbiges Reaktionsprodukt bildet, kolorimetrisch ermittelt und zur Feststellung der Eignung der Milch in einer Kontrollprobe nach Abbau des Harnstoffes mittels Urease den Blindwert mit einem Aldehyd, der außer mit Harnstoff auch mit anderen amino-bzw. amidgruppenhaltigen Verbindungen in einer Schiff'schen Base-Reaktion  ein farbiges Reaktionsprodukt bildet, kolorimetrisch ermittelt.

2. Verfahren nach Anspruch 1, dadurch g e k e n n z e i c h n e t , daß man die Harnstoffbestimmung der Milch in einem Eintopfverfahren durchführt.

3. Verfahren nach Anspruch 1, dadurch g e k e n n z e i c h net, daß man die Harnstoffbestimmung der Milch auf Papier oder einem sonstigen festen Träger durchführt.